# FASCICULE DE BREVET EUROPEEN

(11) **EP 2 560 692 B1**
(45) Date de publication et mention de la délivrance du brevet: **02.01.2019**
(21) Numéro de dépôt: 11711611.1
(22) Date de dépôt: 11.02.2011
(51) Int. Cl.: A61L 2/26, A61L 2/20

(54) **EMBALLAGE BIOPHARMACEUTIQUE STÉRILISABLE**
STERILISIERBARE BIOPHARMAZEUTISCHE VERPACKUNG
STERILIZABLE BIOPHARMACEUTICAL PACKAGING

(30) Priorité: 20.04.2010 FR 1052970
(43) Date de publication de la demande: 27.02.2013
(73) Titulaire: SARTORIUS STEDIM FMT SAS, 13400 Aubagne (FR)
(72) Inventeur: GAY, Isabelle, F-13124 Peypin (FR); NODIN, Gaëlle, F-83470 Saint Maximin La Sainte Baume (FR); MENDYK, Nicolas, F-13124 Peypin (FR); ARMAU, Stéphanie, F-65380 Ossun (FR)
(74) Mandataire: Derambure, Christian
(86) Numéro de dépôt international: PCT/FR2011/050291
(87) Numéro de publication internationale: WO 2011/131870

(56) Documents cités:
- EP-A1- 1 520 795
- WO-A2-03/068274
- US-A- 4 296 862
- US-A- 4 583 643

## Description

L'invention est relative aux emballages biopharmaceutiques stérilisables et vise plus particulièrement un emballage biopharmaceutique stérilisable à l'intérieur, un procédé de mise en oeuvre d'un tel emballage.

On entend ici par biopharmaceutique, ce qui est relatif à la biotechnologie ou à la pharmacie.

On sait que l'on est conduit, dans le domaine biopharmaceutique, à devoir emballer des contenus biopharmaceutiques amenés à l'état stérile, dans des emballages souples à usage unique, à des fins de stockage, transport, manipulation, transfert par exemple dans ou depuis des enceintes stériles. Des contenus biopharmaceutiques tels que ceux ici concernés sont à l'état solide et aptes à être manipulés et transférés. Il peut s'agir par exemple d'un objet stérile tel qu'un récipient, un élément de récipient tel qu'un bouchon, une seringue, mais aussi des éléments de contrôle d'environnement utilisés dans le cadre de processus biopharmaceutiques.

On connaît déjà un premier type d'emballage biopharmaceutique stérilisable à l'intérieur, apte à enfermer un tel contenu biopharmaceutique à stériliser comprenant une paroi extérieure souple composé d'une partie étanche aux gaz et aux germes pathogènes et d'une partie perméable à un gaz de stérilisation dans le sens extérieur vers intérieur (à savoir le sens allant de l'extérieur vers l'intérieur de l'emballage). Avant stérilisation, l'emballage stérilisable comporte une ouverture d'introduction du contenu biopharmaceutique à stériliser depuis l'extérieur vers l'intérieur de l'emballage, cette ouverture d'introduction étant alors à l'état ouvert. Cette ouverture d'introduction est apte à être fermée de façon étanche une fois le contenu biopharmaceutique amené dans l'espace intérieur de réception de l'emballage. Il est prévu qu'après stérilisation, et au moment où cela est souhaité, l'emballage stérilisé comporte une ouverture d'extraction du contenu biopharmaceutique stérilisé, cette ouverture d'extraction étant alors à l'état ouvert, de sorte à dégager le contenu biopharmaceutique de l'emballage en vue de l'emploi souhaité. Précédemment, cette ouverture d'extraction était fermée de façon étanche ou était inexistante. Il est également prévu que la partie de paroi extérieure perméable aux gaz de stérilisation soit apte à être associée à, c'est-à-dire mise en communication avec, une source appropriée de gaz de stérilisation, par exemple le gaz de stérilisation d'une enceinte de stérilisation, de sorte que le gaz de stérilisation provenant de la source en question soit en mesure de pénétrer à l'intérieur de l'emballage par cette partie perméable. Pour la mise en oeuvre d'un tel emballage, on dispose le contenu biopharmaceutique à stériliser dans l'espace intérieur de réception de l'emballage destiné à l'enfermer, via l'ouverture d'introduction précédemment ouverte et ultérieurement scellée de façon étanche. Puis, on injecte le gaz de stérilisation dans l'espace intérieur de réception où se trouve le contenu biopharmaceutique, via la partie perméable au gaz de stérilisation, ce qui assure ainsi la stérilisation recherchée. Au moment souhaité après stérilisation, on ouvre l'ouverture d'extraction du contenu biopharmaceutique stérilisé, de manière à l'extraire de l'emballage afin de l'employer comme souhaité.

De manière non limitative, on entend ici par stérilisation les procédés de stérilisation par la vapeur d'eau saturée, par la chaleur sèche ou encore par des gaz toxiques tels que l'oxyde d'éthylène (ETO), la vapeur-formaldéhyde ou encore le peroxyde d'hydrogène (H2O2). Lors de la stérilisation du contenu biopharmaceutique, l'ambiance enveloppant le contenu biopharmaceutique à stériliser et l'espace intérieur de réception de l'emballage qui enferme le contenu biopharmaceutique à stériliser sont emplis de gaz de stérilisation approprié, de sorte à détruire, dans une certaine mesure souhaitée ou dans la mesure requise, les germes pathogènes.

On entend par fermeture étanche, une fermeture étanche aux gaz, y compris la vapeur d'eau, et aux germes pathogènes.

On entend par partie perméable au gaz de stérilisation, la faculté d'un organe de séparation telle qu'une paroi, y compris une paroi souple telle qu'une membrane, de permettre le passage, au moins dans un sens, d'un gaz de stérilisation approprié, y compris sous forme de vapeur, telle que vapeur d'eau.

Dans une première réalisation, la stérilisation est réalisée sur le site d'emploi du contenu biopharmaceutique, ce contenu ainsi emballé et stérilisé étant alors prêt à l'emploi et devant être employé peu de temps après la stérilisation, en espérant que l'intégrité de l'emballage ait été préservée. En effet, avec une telle réalisation dans laquelle la partie perméable demeure perméable et en contact avec l'atmosphère ambiante, même après stérilisation, il n'est pas possible de mettre en oeuvre un processus de contrôle d'intégrité de l'emballage, du fait de la présence de cette partie perméable. Pourtant, avec une telle réalisation, un contrôle d'intégrité serait d'autant plus nécessaire que le risque de détérioration de la partie perméable après stérilisation est élevé, notamment en cas de transport ou de manipulation.

Dans une seconde réalisation, on assure un contrôle d'intégrité en plaçant l'emballage enfermant le contenu biopharmaceutique stérilisé dans une poche de suremballage, une pression différentielle étant créée entre l'intérieur même de l'emballage et la partie intérieure de la poche de suremballage extérieure à l'emballage. Cette seconde réalisation a pour inconvénient de nécessiter des manipulations compliquées et une durée opératoire importante pour pouvoir détecter un changement de pression différentielle significatif d'une perte d'intégrité.

On sait que dans le domaine biopharmaceutique, il est indispensable de s'assurer que les poches souples soient étanches, du moins présentent un degré d'étanchéité jugé satisfaisant. Il existe plusieurs procédés de contrôle d'intégrité de telles poches.

La norme F 2095 - 01 de ASTM International dont le titre est « Standard Test Methods for Pressure Decay Leak Test for Nonporous Flexible Packages With and Without Restraining Plates » porte plus précisément sur le procédé dit à chute de pression. Ce procédé est envisagé selon deux modes d'exécution possibles : avec plaques de limitation d'expansion ou sans de telles plaques. Le document WO 2009/095572 porte sur un procédé de contrôle d'intégrité dans le cas d'une poche à soufflets latéraux.

On sait également que dans le domaine biopharmaceutique, il est commun d'utiliser soit des poches dont les deux grandes parois sont directement réunies l'une à l'autre de sorte qu'une fois expansées, elles ont un volume limité et restent relativement peu épaisses, soit des poches à soufflets de sorte qu'une fois expansées, elles ont un volume pouvant être beaucoup plus important.

Par conséquent, avec le premier type d'emballage biopharmaceutique stérilisable précédemment décrit, on rencontre le problème du contrôle d'intégrité de l'emballage, avec les conséquences induites : nécessité de réaliser la stérilisation sur le site d'emploi du contenu biopharmaceutique, nécessité d'employer rapidement le contenu emballé et stérilisé, ou encore réalisation d'un contrôle d'intégrité complexe et long.

Un deuxième type d'emballage correspondant à un état de la technique plus proche est décrit dans le document US-A-7040485. Ici, l'emballage comprend une paroi extérieure souple, étanche aux gaz et aux germes pathogènes ; une ouverture d'introduction depuis l'extérieur vers l'intérieur de l'emballage du contenu biopharmaceutique à stériliser, apte par la suite à être fermée de façon étanche ; un espace intérieur apte à recevoir le contenu biopharmaceutique ; un passage d'amenée de gaz de stérilisation depuis l'extérieur de l'emballage, comprenant au moins un orifice d'amenée amont de gaz de stérilisation, vers l'extérieur de l'emballage, apte à être associé en communication avec une source de gaz de stérilisation, au moins un orifice de distribution aval de gaz de stérilisation débouchant dans l'espace intérieur, et une communication entre le au moins un orifice d'amenée amont et le au moins un orifice de distribution aval ; et, enfin, des moyens de commande de la distribution de gaz de stérilisation dans l'espace intérieur.

Dans ce deuxième type d'emballage, la paroi extérieure est une gaine comportant un premier tronçon et un second tronçon adjacents à l'endroit d'un côté qui est ouvert avant stérilisation et fermé lors de la stérilisation. Le premier tronçon de gaine comporte sur l'une de ses deux faces une partie perméable au gaz de stérilisation appartenant à un passage d'amenée de gaz de stérilisation depuis l'extérieur de l'emballage. Le premier tronçon de gaine comporte également une ouverture d'extrémité d'introduction dans l'emballage du contenu biopharmaceutique à stériliser, alors à l'état ouvert. Cette ouverture d'introduction est située à l'opposé du côté ouvert. Le second tronçon de gaine est fermé sur son pourtour, à l'exception du côté ouvert avant stérilisation et fermé lors de la stérilisation. Le second tronçon de gaine forme un espace intérieur de réception du contenu biopharmaceutique à stériliser. L'emballage comporte également des moyens de commande de la distribution de gaz de stérilisation dans l'espace intérieur pour que soit il accède soit il ne puisse accéder à l'ambiance enveloppant le contenu à stériliser et à l'espace intérieur de l'emballage qui l'enferme. Pour la mise en oeuvre d'un tel emballage, on introduit le contenu biopharmaceutique à stériliser dans l'emballage par passage à travers l'ouverture d'extrémité d'introduction, puis par traversée du premier tronçon à savoir passage devant le tronçon perméable au gaz de stérilisation et passage à travers le côté ouvert. C'est ainsi que le contenu biopharmaceutique accède à l'espace intérieur de réception dans lequel il est placé pour la stérilisation. L'ouverture d'extrémité d'introduction est alors scellée sur elle-même et ainsi fermée, tandis que le côté est maintenu ouvert. Le gaz de stérilisation est alors introduit dans l'emballage, depuis l'extérieur de l'emballage, à travers la partie perméable. Ainsi, le gaz de stérilisation pénètre dans le premier tronçon de gaine puis, passant à travers le côté ouvert, pénètre dans le second tronçon de gaine. On assure ainsi la stérilisation du contenu biopharmaceutique. Le côté ouvert est alors scellé sur lui-même et ainsi fermé. L'emballage est alors scindé en deux, les deux tronçons de gaine étant séparés, la gaine étant coupée transversalement vers le côté ouvert qui a été précédemment scellé.

Ainsi, avec ce deuxième type d'emballage, le côté ouvert sert à la fois à l'introduction du contenu biopharmaceutique à stériliser et au passage d'amenée de gaz de stérilisation. Par suite, le côté ouvert attenant à l'espace intérieur ne peut être fermée indépendamment et avant la fermeture du passage d'amenée de gaz de stérilisation.

D'autre part, l'ouverture d'introduction du contenu biopharmaceutique à stériliser est écartée de l'espace intérieur de réception du contenu biopharmaceutique à stériliser et du contenu biopharmaceutique stérilisé, de sorte que le contenu biopharmaceutique à stériliser n'est introduit dans l'espace intérieur depuis l'extérieur de l'emballage que de façon indirecte. Une telle réalisation est donc problématique à mettre en oeuvre dans certains cas, compte tenu de la forme ou de la taille de l'emballage ou de la nature, de la forme et de la taille du contenu biopharmaceutique.

On rencontre le même inconvénient avec les emballages décrits dans les documents WO 2004/039419 et FR-A-2820118.

Egalement, les moyens de commande de la distribution de gaz de stérilisation sont des moyens d'ouverture/fermeture du côté qui participe à l'introduction du contenu à stériliser dans l'emballage, de sorte que le passage d'amené de gaz de stérilisation ne peut pas être ouvert ou fermé indépendamment de l'ouverture ou de la fermeture de l'introduction du contenu à stériliser.

Enfin, une fois la stérilisation faite, la paroi extérieure, le passage d'amenée de gaz de stérilisation et les moyens d'ouverture/fermeture sont nécessairement séparés en deux. Compte tenu que dans l'emballage stérilisé, il n'y a plus de partie perméable, celle-ci ayant été enlevée, il est possible de mettre en oeuvre un processus de contrôle d'intégrité de l'emballage dont la paroi est prévue étanche de fabrication et dont il est attendu qu'elle demeure étanche. Toutefois, cette possibilité de mettre en oeuvre un processus de contrôle d'intégrité n'est obtenue qu'au prix d'une section de l'emballage, pour séparer les deux tronçons de gaine. Or, une telle opération de section de la gaine est toujours délicate car, précisément, elle peut compromettre l'intégrité de l'emballage.

Par conséquent, avec le second type d'emballage biopharmaceutique stérilisable précédemment décrit, on rencontre le problème de la difficulté de faire passer le contenu biopharmaceutique dans l'espace intérieur et le problème du risque de détérioration de l'intégrité de l'emballage par suite de sa section.

Il est également connu de l'état de la technique, le document US 4,583,643 décrivant un emballage flexible destiné à recevoir des articles pouvant être automatiquement stérilisé de manière à assurer leur intégrité. L'emballage flexible présente un premier module formant valve et actionneur positionné sur la paroi supérieure et un deuxième module formant valve et actionneur positionné sur la paroi inférieure. Chacun de ces modules formant valve et actionneur est doté d'une pièce de support comportant une pluralité de trous faisant office d'ouvertures afin de permettre le passage d'un écoulement fluide à l'intérieur et à l'extérieur de l'emballage flexible et d'une valve utilisée pour refermée les trous.

Il est connu le document US 4,296,862 décrivant une pièce destinée à constituer une barrière stérile afin de maintenir l'intérieur d'un dispositif d'emballage dans des conditions stériles.

Il est aussi connu le document WO 03/068274 se rapportant à un système permettant d'injecter du gaz ozonisé à l'intérieur d'un emballage contenant un objet à désinfecter ou à stériliser. L'emballage comporte des moyens de couplage lui permettant de s'associer audit système d'injection pour assurer l'introduction du gaz ozonisé à l'intérieur de la chambre recevant l'objet à stériliser, ainsi que des moyens de fermeture permettant d'assurer l'étanchéité de la chambre lorsque l'emballage n'est plus associé au système.

Il est, par ailleurs, connu le document EP1 520 795 qui décrit un emballage pour produits pharmaceutiques formé à partir d'un premier feuillet imperméable, d'un second feuillet imperméable soudé au premier et d'un fermoir perméable soudé au second feuillet et susceptible d'être soudé au premier feuillet. Ces trois feuillets forment un espace intérieur dans lequel peut être introduit un objet en vue de sa stérilisation. Lorsque ladite opération de stérilisation est terminée, la portion de l'emballage contenant le fermoir perméable est sectionnée et les premier et second feuillets imperméables sont soudés l'un à l'autre de manière à constituer un espace intérieur délimité uniquement par ces premier et second feuillets imperméables. Comme précédemment, une telle opération de section est périlleuse car elle peut compromettre l'intégrité de l'emballage.

L'invention a donc pour but, dans le cas d'un emballage biopharmaceutique stérilisable apte à enfermer un contenu biopharmaceutique à stériliser, à pallier les inconvénients et limites des emballages connus et à apporter une solution, en combinaison, aux trois problèmes suivants :
▪ possibilité d'introduire de façon simple le contenu biopharmaceutique directement dans l'espace intérieur de l'emballage sans nécessité de traverser un autre compartiment,
▪ possibilité de ne pas devoir sectionner l'emballage une fois la stérilisation réalisée,
▪ possibilité de réaliser un contrôle d'intégrité de l'emballage avec pression différentielle qui soit rapide et puisse intervenir à tout moment dès la stérilisation, en tout cas n'a pas obligatoirement à intervenir sur le site d'emploi du contenu biopharmaceutique et juste avant l'emploi.

L'invention a également pour but d'atteindre ces objectifs avec des moyens simples, peu coûteux, et faciles à mettre en oeuvre.

A cet effet, et selon un premier aspect, l'invention a pour objet un emballage biopharmaceutique stérilisable, apte à enfermer un contenu biopharmaceutique à stériliser, telle que définie dans la revendication 1.

Un tel emballage est tel que :
▪ l'ouverture d'introduction du contenu biopharmaceutique à stériliser est distincte du passage d'amenée de gaz de stérilisation, de sorte que l'ouverture d'introduction du contenu biopharmaceutique à stériliser peut être fermée indépendamment et avant la fermeture du passage d'amenée de gaz de stérilisation,
▪ l'ouverture d'introduction du contenu biopharmaceutique à stériliser est directement adjacente à l'espace intérieur de réception du contenu biopharmaceutique à stériliser et du contenu biopharmaceutique stérilisé, de sorte que le contenu biopharmaceutique à stériliser est introduit directement dans l'espace intérieur depuis l'extérieur de l'emballage,
▪ les moyens de commande de la distribution de gaz de stérilisation sont des moyens d'ouverture/fermeture du passage d'amenée de gaz de stérilisation, de sorte que le passage d'amené de gaz de stérilisation peut être ouvert ou fermé indépendamment de l'ouverture ou fermeture de l'ouverture d'introduction,
▪ une fois la stérilisation faite, la paroi extérieure, le passage d'amenée de gaz de stérilisation et les moyens d'ouverture/fermeture sont aptes à rester solidaires entre eux de sorte à former un emballage biopharmaceutique stérilisé enfermant le contenu biopharmaceutique stérilisé pouvant subir un ou plusieurs tests d'intégrité à tout moment souhaité après la stérilisation.

Avec un tel emballage, il est possible d'introduire de façon simple le contenu biopharmaceutique directement dans l'espace intérieur de l'emballage sans nécessité de traverser un autre compartiment, il est possible de ne pas devoir sectionner l'emballage une fois la stérilisation réalisée, et, enfin, il est également possible de réaliser un contrôle d'intégrité de l'emballage avec pression différentielle qui soit rapide et puisse intervenir à tout moment dès la stérilisation, en tout cas n'a pas obligatoirement à intervenir sur le site d'emploi du contenu biopharmaceutique et juste avant l'emploi.

Selon une première réalisation, le passage d'amenée de gaz de stérilisation inclut au moins une membrane perméable au gaz de stérilisation, surfaciquement étendue, formant un orifice de distribution aval de gaz de stérilisation distribué sur la surface de la membrane, la membrane perméable au gaz de stérilisation formant une partie de l'enveloppe de l'espace intérieur constituant également un moyen apte à retenir d'éventuels germes pathogènes.

Selon un mode d'exécution, une membrane perméable au gaz de stérilisation est constituée par une zone perméable de la paroi extérieure de l'emballage appartenant à une partie déployable/pliable de la paroi extérieure de l'emballage et formant le passage d'amenée de gaz de stérilisation, cette partie déployable/pliable étant apte à être soit déployée de sorte que le passage d'amenée de gaz de stérilisation est ouvert soit pliée sur elle-même vers l'intérieur de l'emballage en sorte que le passage d'amenée de gaz de stérilisation soit fermé.

Selon les cas, la partie de la paroi extérieure de l'emballage apte à être soit à l'état déployé soit à l'état plié sur elle-même vers l'intérieur est un soufflet longitudinal de la paroi extérieure ou une partie transversale d'extrémité de la paroi extérieure.

Selon une réalisation, les moyens d'ouverture/fermeture sont constitués par la paroi extérieure elle-même, dont la partie déployable/pliable où se trouve la zone perméable au gaz de stérilisation, est interposée entre deux parties collatérales étanches situées de part et d'autre de la partie déployable/pliable, de manière telle que lorsque la partie déployable/pliable est pliée sur elle-même vers l'intérieur de l'emballage, les deux parties collatérales, alors situées en vis-à-vis et à proximité l'une de l'autre, sont aptes à être scellées l'une à l'autre à leur périphérie extérieure de façon étanche, autour de la zone perméable au gaz de stérilisation, de sorte à empêcher le gaz de stérilisation d'accéder à zone perméable.

Selon une réalisation, le passage d'amenée de gaz de stérilisation inclut au moins un tronçon de tube d'amenée de gaz de stérilisation, situé à l'extérieur de l'emballage, formant vers l'amont un orifice d'amenée amont de gaz de stérilisation et vers l'aval un orifice de distribution aval de gaz de stérilisation ménagé dans la paroi extérieure, le tronçon de tube d'amenée de gaz de stérilisation étant solidaire de la paroi extérieure de façon étanche, au moins un moyen apte à retenir d'éventuels germes pathogènes, tel qu'une cartouche filtrante, étant interposé sur le tronçon de tube d'amenée de gaz de stérilisation entre l'orifice d'amenée amont et l'orifice de distribution aval.

Dans ce cas, selon une réalisation, les moyens d'ouverture/d'obturation du passage d'amenée de gaz de stérilisation, sont un moyen d'arrêt de flux monté mobile ou amovible sur le tronçon de tube d'amenée de gaz de stérilisation, notamment une pince de fermeture ou un soudage transversal.

Selon une réalisation, l'espace intérieur s'étend sur substantiellement toute la longueur de la paroi extérieure en forme de gaine, et l'ouverture d'introduction du contenu biopharmaceutique à stériliser est située à une extrémité de la paroi extérieure en forme de gaine.

Selon une réalisation, le au moins un orifice d'amenée amont de gaz et l'ouverture d'introduction du contenu biopharmaceutique à stériliser sont écartés l'un de l'autre.

Selon un développement, l'emballage comporte également au moins un embout d'entrée/sortie de gaz, monté sur la paroi extérieure, en communication avec l'espace intérieur, apte à être amené à l'état ouvert ou à l'état fermé par actionnement de moyens d'obturation, de sorte à permettre de faire entrer dans ou de faire sortir de l'espace intérieur, un gaz fonctionnel.

Selon une réalisation, l'emballage biopharmaceutique comporte également une ouverture délimitée par une bride annulaire sur laquelle est monté un moyen mobile ou amovible de transfert aseptique, tel qu'une porte mobile ou amovible de transfert aseptique de type à double porte.

Dans ce cas, selon une première variante de réalisation, le moyen mobile ou amovible de transfert aseptique coopère avec l'ouverture d'introduction et est apte à assurer la fermeture de cette ouverture d'introduction de façon étanche.

Dans ce cas, selon une seconde variante de réalisation, le moyen mobile ou amovible de transfert aseptique coopère avec une portion de la paroi extérieure distincte de la membrane perméable au gaz de stérilisation de manière à former une ouverture secondaire apte à être ouverte puis fermée de façon étanche.

Selon un deuxième aspect, un procédé de mise en oeuvre d'un emballage biopharmaceutique stérilisable tel qu'il a été précédemment décrit, est décrit en vue d'y enfermer et d'y stériliser un contenu biopharmaceutique, dans lequel :
▪ on dispose d'un tel emballage, vide du contenu biopharmaceutique, et dont l'ouverture d'introduction est ouverte,
▪ on dispose du contenu biopharmaceutique à stériliser,
▪ on dispose d'une source de gaz de stérilisation,
▪ via l'ouverture d'introduction, on introduit le contenu biopharmaceutique à stériliser directement dans l'espace intérieur de réception,
▪ puis, on ferme l'ouverture d'introduction de façon étanche,
▪ puis, alors que le passage d'amenée de gaz de stérilisation est ouvert, on amène le gaz de stérilisation depuis la source de gaz de stérilisation jusque dans l'espace intérieur de réception où se trouve le contenu biopharmaceutique à stériliser, de sorte à stériliser le contenu biopharmaceutique,
▪ puis, on met en oeuvre les moyens d'ouverture/fermeture de sorte à fermer le passage d'amenée de gaz de stérilisation,
et on forme ainsi un emballage biopharmaceutique stérilisé enfermant le contenu biopharmaceutique stérilisé pouvant subir un ou plusieurs tests d'intégrité à tout moment souhaité après la stérilisation.

Selon une réalisation, on dispose d'un emballage biopharmaceutique tel que décrit, avec une membrane perméable au gaz de stérilisation constituée par une zone perméable de la paroi extérieure de l'emballage appartenant à une partie déployable/pliable, et on dispose de moyens d'ouverture/fermeture constitués par la paroi extérieure dont la partie déployable/pliable est interposée entre deux parties collatérales étanches. Dans ce cas, après que le contenu biopharmaceutique a été stérilisé, on plie la partie déployable/pliable sur elle-même vers l'intérieur de l'emballage, de sorte que les deux parties collatérales soient situées en vis-à-vis et à proximité l'une de l'autre, et on les scelle l'une à l'autre à leur périphérie extérieure de façon étanche, autour de la zone perméable au gaz de stérilisation, de sorte à fermer le passage d'amenée de gaz de stérilisation.

Selon une réalisation, on dispose d'un emballage biopharmaceutique tel que décrit, avec un tronçon de tube d'amenée de gaz de stérilisation, et on dispose de moyens d'ouverture/d'obturation du passage d'amenée de gaz de stérilisation étant un moyen d'arrêt de flux. Dans ce cas, après que le contenu biopharmaceutique a été stérilisé, on interpose le moyen d'arrêt de flux sur le tronçon de tube d'amenée de gaz de stérilisation, de sorte à fermer le passage d'amenée de gaz de stérilisation.

Selon une réalisation, une fois la stérilisation faite, on garde solidaires la paroi extérieure, le passage d'amenée de gaz de stérilisation et les moyens d'ouverture/fermeture de sorte à former l'emballage biopharmaceutique stérilisé enfermant le contenu biopharmaceutique stérilisé.

Selon une caractéristique, une fois la stérilisation faite, on ne sectionne pas l'emballage biopharmaceutique en plusieurs tronçons.

Selon un développement, à tout moment souhaité après la stérilisation, on fait subir à l'emballage biopharmaceutique stérilisé enfermant le contenu biopharmaceutique stérilisé un ou plusieurs tests d'intégrité du type requérant une étanchéité au gaz de l'emballage.

Selon un troisième aspect, l'invention a pour objet un emballage biopharmaceutique stérilisé enfermant un contenu biopharmaceutique stérilisé, obtenu par la mise en oeuvre du procédé tel que décrit, l'emballage comportant également une ouverture d'extraction depuis l'intérieur vers l'extérieur de l'emballage le contenu biopharmaceutique stérilisé, fermée de façon étanche et apte par la suite à être ouverte en sorte d'extraire le contenu biopharmaceutique de l'espace intérieur.

On décrit maintenant plusieurs modes de réalisation de l'emballage à l'aide des dessins, dans lesquels :

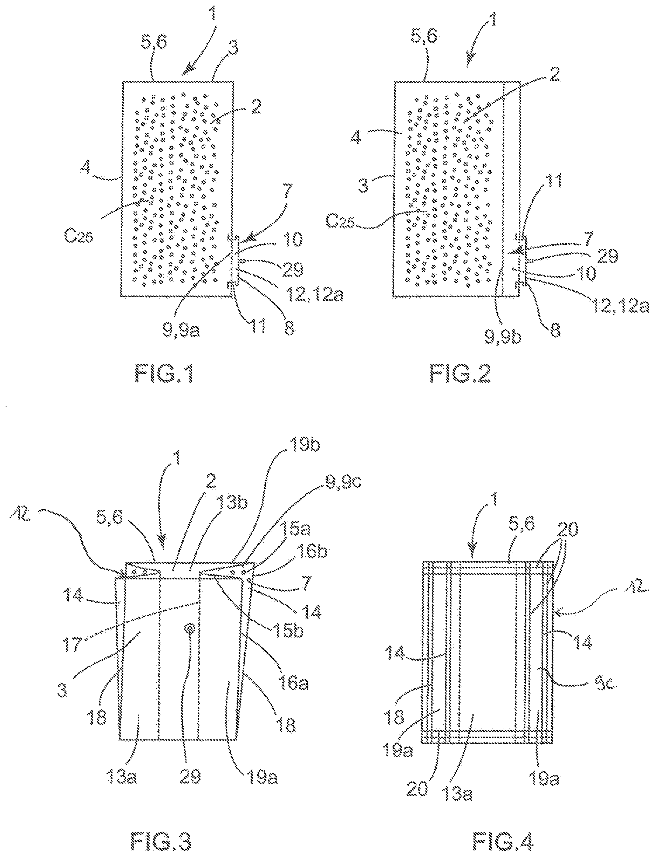

- La figure 1 est une vue en élévation d'un emballage biopharmaceutique stérilisable dont l'espace intérieur est empli d'un contenu biopharmaceutique à stériliser, ayant un passage d'amenée de gaz de stérilisation depuis l'extérieur de l'emballage incluant une membrane perméable au gaz de stérilisation distincte de la paroi extérieure de l'emballage et rapportée sur elle et solidarisée à elle de façon étanche, et des moyens de commande de la distribution de gaz de stérilisation dans l'espace intérieur ici fermant le passage d'amenée de gaz de stérilisation, l'ouverture d'introduction depuis l'extérieur vers l'intérieur de l'emballage du contenu biopharmaceutique à stériliser étant ici fermée.
- La figure 2 est une vue analogue à la figure 1 d'un emballage biopharmaceutique stérilisable.
- La figure 3 est une vue en élévation et en perspective d'un exemple d'un emballage biopharmaceutique stérilisable ayant un passage d'amenée de gaz de stérilisation depuis l'extérieur de l'emballage incluant une membrane perméable au gaz de stérilisation constituée par une zone perméable de la paroi extérieure de l'emballage appartenant à une partie déployable/pliable de la paroi extérieure de l'emballage qui est un soufflet longitudinal de la paroi
   extérieure, et des moyens de commande de la distribution de gaz de stérilisation dans l'espace intérieur ici ouvrant le passage d'amenée de gaz de stérilisation, l'ouverture d'introduction depuis l'extérieur vers l'intérieur de l'emballage du contenu biopharmaceutique à stériliser étant ici ouverte.

- La figure 4 est une vue en élévation de l'emballage biopharmaceutique de la figure 3, une fois stérilisé, les moyens de commande de la distribution de gaz de stérilisation dans l'espace intérieur ici fermant le passage d'amenée de gaz de stérilisation, l'ouverture d'introduction depuis l'extérieur vers l'intérieur de l'emballage du contenu biopharmaceutique à stériliser étant ici également fermée.

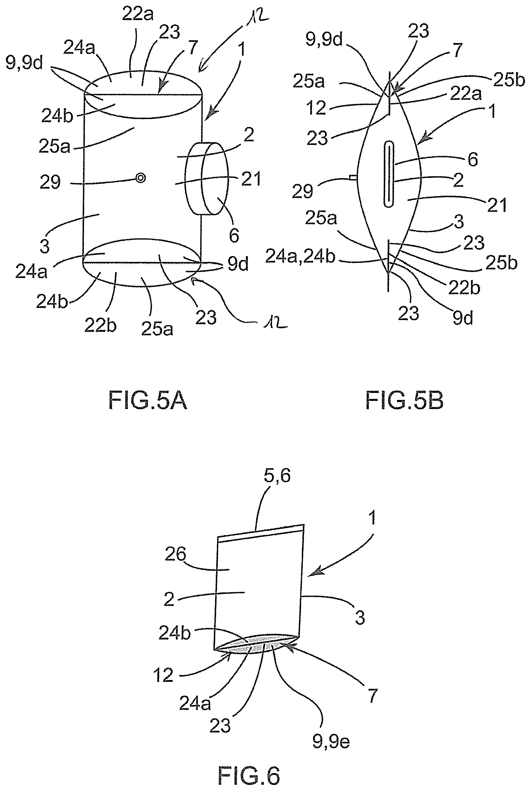

- Les figures 5A et 5B sont deux vues respectivement en élévation et en perspective et en élévation de profil d'un autre exemple d'un emballage biopharmaceutique stérilisable, dans lequel la partie de la paroi extérieure de l'emballage apte à être soit à l'état déployé soit à l'état plié sur elle-même vers l'intérieur est formée de deux parties transversales d'extrémité de la paroi extérieure, les moyens de commande de la distribution de gaz de stérilisation dans l'espace intérieur ouvrant le passage d'amenée de gaz de stérilisation dans le cas de la figure 5A , la partie déployable/pliable de la paroi extérieure de l'emballage étant ici déployée, alors que les moyens de commande de la distribution de gaz de stérilisation dans l'espace intérieur ferment le passage d'amenée de gaz de stérilisation dans le cas de la figure 5B , la partie déployable/pliable de la paroi extérieure de l'emballage étant ici pliée sur elle-même vers l'intérieur de l'emballage.
- La figure 6 est une vue analogue à la figure 5A, la partie déployable/pliable de la paroi extérieure de l'emballage étant ici déployée, alors que les moyens de commande de la distribution de gaz de stérilisation dans l'espace intérieur ouvrent le passage d'amenée de gaz de stérilisation, la partie déployable/pliable de la paroi extérieure de l'emballage étant formée d'une unique partie transversale d'extrémité.

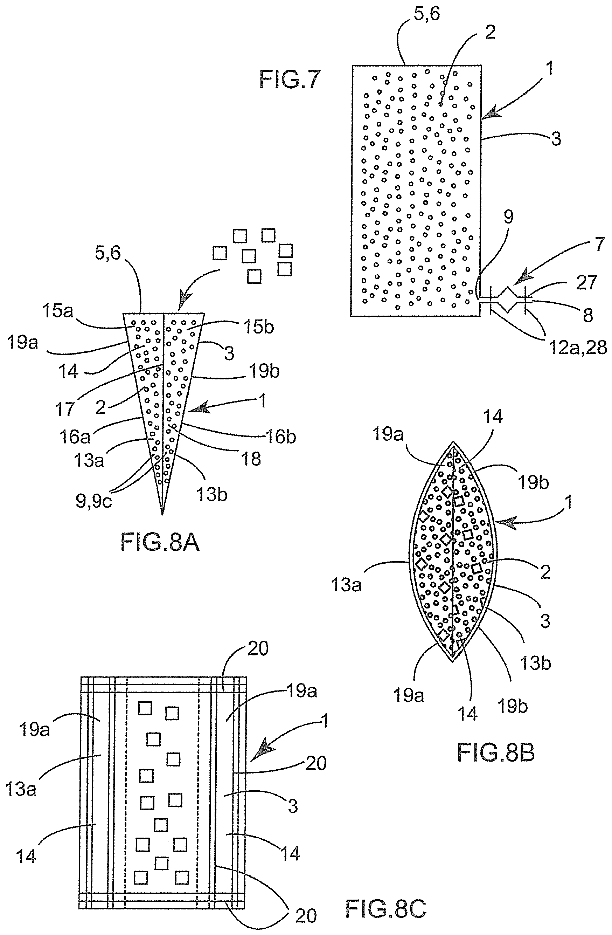

- La figure 7 est une vue en élévation d'un exemple d'un emballage biopharmaceutique stérilisable, ayant un passage d'amenée de gaz de stérilisation depuis l'extérieur de l'emballage incluant un tronçon de tube d'amenée de gaz de stérilisation, situé à l'extérieur de l'emballage, formant vers l'amont un orifice d'amenée amont de gaz de stérilisation et vers l'aval un orifice de distribution aval de gaz de stérilisation ménagé dans la paroi extérieure, le tronçon de tube d'amenée de gaz de stérilisation étant solidaire de la paroi extérieure de façon étanche, un moyen apte à retenir d'éventuels germes pathogènes étant interposé sur le tronçon de tube d'amenée de gaz de stérilisation entre l'orifice d'amenée amont et l'orifice de distribution aval.
- Les figures 8A, 8B et 8C sont trois vues respectivement en élévation illustrant des étapes successives de la mise en oeuvre d'un emballage selon l'exemple des figures 3 et 4, à savoir - avant la stérilisation - l'introduction du contenu à stériliser dans l'espace intérieur de l'emballage par l'ouverture d'introduction ouverte (figure 8A), pour et pendant la stérilisation, l'amenée de gaz de stérilisation dans l'espace intérieur, le passage d'amenée étant ouvert les moyens de commande de la distribution de gaz de stérilisation dans l'espace intérieur ouvrant ce passage (figure 8B), après stérilisation, la fermeture du passage d'amenée de gaz de stérilisation, les deux soufflets longitudinaux de l'emballage stérilisé formant la partie déployable/pliable étant pliés sur eux-mêmes vers l'intérieur de l'emballage et scellés à leur périphérie.

On se réfère à la figure 1 qui illustre un emballage biopharmaceutique stérilisable 1 dont l'espace intérieur 2 est empli d'un contenu biopharmaceutique à stériliser C_{às}.

Bien entendu, l'espace intérieur 2 est adapté, notamment en taille, au contenu biopharmaceutique à stériliser C_{às} qu'il doit recevoir et enfermer.

L'emballage 1 comporte une paroi extérieure 3 souple, étanche aux gaz et aux germes pathogènes, par exemple en matière plastique telle que le polyéthylène ou polypropylène.

Une telle paroi extérieure 3 est par exemple du type formé de deux grandes faces de forme générale rectangulaire, réunies l'une à l'autre à leur périphérie 4, à l'exception d'un bord transversal d'extrémité 5, de sorte à constituer une poche dite « 2D » (D signifiant dimensions), bien connue dans le domaine biopharmaceutique. Un tel emballage 1 est bien adapté à un contenu biopharmaceutique à stériliser C_{às} de taille relativement limitée.

Le bord transversal d'extrémité 5 est originellement à l'état ouvert. Il constitue ainsi et alors une ouverture d'introduction 6 depuis l'extérieur vers l'intérieur de l'emballage 1 du contenu biopharmaceutique à stériliser C_{às}. Par la suite, c'est-à-dire une fois le contenu biopharmaceutique à stériliser C_{às} disposé dans l'espace intérieur 2, l'ouverture d'introduction 6 est fermée de façon étanche, par exemple par scellage, le matériau constitutif de la paroi extérieure 3 étant thermoscellable sur lui-même.

Par suite de la structure qui vient d'être décrite, l'ouverture d'introduction 6 est directement adjacente à l'espace intérieur 2. Dès lors, le contenu biopharmaceutique à stériliser C_{às} peut être introduit directement dans l'espace intérieur 2 depuis l'extérieur E de l'emballage 1.

L'emballage 1 comporte également un passage 7 d'amenée de gaz de stérilisation depuis l'extérieur E de l'emballage 1.

Le passage 7 d'amenée de gaz de stérilisation comprend ici un orifice d'amenée amont 8 de gaz de stérilisation, situé vers l'extérieur E de l'emballage 1. Cet orifice d'amenée 8 est apte à être associé en communication avec une source de gaz de stérilisation, telle que par exemple l'ambiance régnant dans une enceinte de stérilisation.

Le passage 7 d'amenée de gaz de stérilisation comprend également une pluralité d'orifices de distribution aval 9 de gaz de stérilisation débouchant dans l'espace intérieur 2.

Enfin, le passage 7 d'amenée de gaz de stérilisation comprend une communication 10 entre l'orifice d'amenée amont 8 et les orifices de distribution 9.

Le passage d'amenée 7 est distinct de l'ouverture d'introduction 6 et inversement l'ouverture d'introduction 6 est distincte du passage d'amenée 7. Ce faisant, l'ouverture d'introduction 6 peut être fermée indépendamment et avant la fermeture du passage d'amenée 7.

Dans la figure 1, l'orifice d'amenée amont 8 est formé par un orifice ménagé dans la paroi extérieure 3. Cet orifice 8 est limité par une virole 11.

D'autre part, le passage d'amenée 7 inclut une membrane 9a perméable au gaz de stérilisation, laquelle membrane 9a forme la pluralité d'orifices de distribution aval 9.

La membrane 9a est distincte de la paroi extérieure 3. Elle est placée dans l'emballage 1 lui-même. Elle est rapportée sur la paroi extérieure 3 et solidarisée à elle de façon étanche, par exemple par scellage.

Une telle membrane 9a est par exemple réalisée en un matériau tel que celui connu sous la marque TYVEK®. Il s'agit d'un textile non-tissé de fibres de polyéthylène à haute densité qui, après extrusion, sont organisées de façon aléatoire et consolidées, la feuille en résultant étant poreuse de façon sélective, ici au gaz de stérilisation qu'elle doit laisser passer dans le sens allant de l'extérieur vers l'intérieur. Le matériau connu sous la marque TYVEK® n'est pas exclusif d'autres présentant également une perméabilité sélective au gaz de stérilisation.

C'est la porosité et la perméabilité sélective de la membrane 9a qui constituent les orifices de distribution 9 ou l'ensemble des orifices de distribution 9.

Il convient de signaler ici que, dans la mesure où la membrane 9a présente une porosité et une perméabilité sélective vis-à-vis du gaz de stérilisation, cette dernière peut être capable de laisser passer ce gaz de stérilisation non seulement dans le sens allant de l'extérieur vers l'intérieur mais également dans le sens allant de l'intérieur vers l'extérieur. Ainsi, la membrane 9a peut former une pluralité d'orifices de distribution aval 9 permettant, d'une part, l'introduction du gaz de stérilisation dans l'espace intérieur 2 de l'emballage biopharmaceutique 1 et, d'autre part, la sortie de ce même gaz de distribution vers la communication 10 et l'orifice d'amenée amont 8.

L'intérêt d'utiliser une membrane 9a réside donc également dans le fait qu'il n'est pas nécessaire d'utiliser de moyens d'évacuation du gaz de stérilisation distincts et indépendants des moyens d'amenée du gaz de stérilisation à l'issue de l'opération de stérilisation pour extraire le gaz de stérilisation de l'espace intérieur de l'enceinte.

La membrane 9a s'étend substantiellement en regard de l'orifice amont d'amenée 8. La membrane 9a, surfaciquement étendue, forme ainsi, en regard de l'orifice amont d'amenée 8, une partie de l'enveloppe 2a de l'espace intérieur 2. Avec cette structure, la communication 10 forme une sorte de cylindre limité aux extrémités par la membrane 9a et par l'orifice amont d'amenée 8 et dont le diamètre est celui de la membrane 9a et de l'orifice 8.

Une telle membrane 9a permet non seulement de distribuer le gaz de stérilisation à partir de toute la surface de la membrane, mais également constitue un moyen apte à retenir d'éventuels germes pathogènes.

L'emballage 1 comporte également des moyens de commande 12 de la distribution de gaz de stérilisation dans l'espace intérieur 3.

Les moyens de commande 12 de la distribution de gaz de stérilisation sont des moyens d'ouverture/fermeture 12 du passage d'amenée 7, de sorte que le passage d'amené 7 peut être ouvert ou fermé indépendamment de l'ouverture ou fermeture de l'ouverture d'introduction 6.

En l'espèce, les moyens d'ouverture/fermeture 12 se présentent sous la forme d'un organe de bouchage 12a monté mobile ou amovible sur la virole 11. Un tel organe de bouchage 12a peut être constitué par une pièce en forme de paroi étanche fixée sur la virole au moyen d'un système de fermeture tel que celui connu sous la dénomination et la marque TRI-CLAMP® ou tout autre système de fixation.

Le procédé de mise en oeuvre de l'emballage 1 qui vient d'être décrit est destiné à y enfermer un contenu biopharmaceutique à stériliser C_{às} et à le stériliser.

Dans ce procédé, on dispose d'un tel emballage 1, avec une membrane 9a perméable au gaz de stérilisation distincte de la paroi extérieure 3, vide du contenu biopharmaceutique à stériliser C_{às}, et dont l'ouverture d'introduction 6 est ouverte. On dispose de moyens d'ouverture/fermeture 12, ici l'organe de bouchage 12a. On dispose également d'une source de gaz de stérilisation. On dispose enfin du contenu biopharmaceutique à stériliser C_{às} à stériliser.

Via l'ouverture d'introduction 6, on introduit le contenu biopharmaceutique à stériliser C_{às} directement dans l'espace intérieur de réception 2.

Puis, on ferme l'ouverture d'introduction 6 de façon étanche, en scellant le bord transversal 5 jusqu'ici ouvert.

Puis, alors que le passage d'amenée 7 est ouvert, pour la raison que l'organe de bouchage 12a constitutifs des moyens d'ouverture/fermeture 12 sont enlevés, en tout cas inactifs au regard de la fonction de bouchage, on amène le gaz de stérilisation depuis la source de gaz de stérilisation jusque dans l'espace intérieur 2 où se trouve le contenu biopharmaceutique à stériliser C_{às}. C'est ainsi que l'on stérilise le contenu biopharmaceutique.

Le gaz de stérilisation peut alors être retiré de l'espace intérieur 2 en passant au travers du passage 7 d'amenée de gaz de stérilisation qui constitue alors un passage d'évacuation du gaz de stérilisation vers la communication 10, l'orifice d'amenée amont 8 et l'extérieur.

De contenu biopharmaceutique à stériliser C_{às}, il devient contenu biopharmaceutique stérilisé Cₛ.

Une fois la stérilisation faite, on met en oeuvre l'organe de bouchage 12a de sorte à fermer le passage d'amenée 7 et, à cet effet, on le place sur la virole 11 de l'orifice amont d'amenée 8 de gaz de stérilisation.

On a alors formé un emballage biopharmaceutique 1 stérilisé enfermant le contenu biopharmaceutique stérilisé Cₛ.

L'emballage biopharmaceutique 1 stérilisé est constitué de la paroi extérieure 3, le passage 7 d'amenée de gaz de stérilisation et les moyens d'ouverture/fermeture 12, en l'espèce l'organe de bouchage 12a, qui restent solidaires entre eux et enferment le contenu biopharmaceutique stérilisé Cₛ.

Avec cette disposition, cet emballage biopharmaceutique 1 stérilisé et le contenu biopharmaceutique stérilisé Cₛ peuvent subir un ou plusieurs tests d'intégrité à tout moment souhaité après la stérilisation, soit immédiatement ou peu de temps après fabrication, soit immédiatement ou peu de temps avant l'emploi, soit encore à plusieurs moments.

On se réfère à la figure 2 qui illustre un emballage biopharmaceutique stérilisable 1.

Contrairement à la variante de la figure 1, la membrane 9b perméable au gaz de stérilisation s'étend sur substantiellement toute la longueur de la paroi extérieure 3 de l'emballage 1, et non seulement en regard de l'orifice amont d'amenée 8. Avec cette structure, la communication 10 s'étend sur substantiellement toute la longueur de la paroi extérieure 3 vers la membrane 9a et sur le diamètre de l'orifice amont d'amenée 8, vers ce dernier. En outre, la membrane 9b est écartée en regard d'une partie correspondante de la paroi extérieure 3 de sorte à former une sorte de couloir permettant au gaz de stérilisation d'alimenter la totalité de la surface de la membrane 9a perméable.

Cette disposition constructive permet de bien répartir le gaz de stérilisation qui pénètre directement dans l'espace intérieur 2 sur une grande surface de son enveloppe.

La mise en oeuvre d'un tel emballage 1 à membrane 9b perméable au gaz de stérilisation est analogue à celle décrite précédemment pour l'emballage 1 à membrane 9a perméable au gaz de stérilisation.

On se réfère aux figures 3, 4, 5A, 5B, 6, 8A, 8B et 8C qui illustrent, un mode d'exécution dans lequel la membrane est constituée par une zone perméable de la paroi extérieure 3 de l'emballage 1.

On se réfère plus spécialement aux figures 3, 4, 8A, 8B et 8C qui illustrent une première variante de ce mode d'exécution.

Dans cette variante, la paroi extérieure 3 comprend deux grandes faces 13a et 13b, ici de forme générale rectangulaire, réunies l'une à l'autre par deux soufflets longitudinaux 14, chacun comportant deux volets 15a et 15b, attenant aux deux grandes faces 13a et 13b par deux lignes de pliage 16a et 16b latérales, tandis que les deux volets 15a et 15b sont attenants l'un à l'autre par une ligne de pliage médiane 17.

Les deux volets 15a et 15b d'un même soufflet longitudinal 14 ont une même largeur transversale inférieure à la largeur transversale des grandes faces 13a et 13b. D'autre part, les deux volets 14 sont situés vers l'intérieur de l'emballage, c'est-à-dire que les deux volets 15a et 15b et la ligne de pliage médiane 17 sont situés en regard des deux grandes faces 13a et 13b, lorsque l'emballage 1 est plié à plat.

Une telle structure, ou des structures différentes mais analogues, permettent de constituer des poches à soufflets pouvant être pliées à plat ou dépliées déployées.

II est prévu une membrane 9c perméable au gaz de stérilisation qui appartient à une partie déployable/pliable 18 de la paroi extérieure 3 de l'emballage 1, à savoir plus précisément les soufflets longitudinaux 14, la partie déployable/pliable 18 étant ici double. Bien entendu, il peut être prévu une partie déployable/pliable 18 simple.

La membrane 9c forme le passage 7 d'amenée de gaz de stérilisation.

Comme indiqué, la partie déployable/pliable 18, c'est-à dire les soufflets longitudinaux 14, formant la membrane 9c, est apte à être soit déployée de sorte que le passage 7 d'amenée de gaz de stérilisation est ouvert soit pliée sur elle-même vers l'intérieur de l'emballage 1, en sorte que le passage 7 d'amenée de gaz de stérilisation soit alors fermé.

Avec cette variante d'emballage 1, les moyens de commande 12 de la distribution de gaz de stérilisation sont, ici aussi, des moyens d'ouverture/fermeture 12 du passage d'amenée 7, mais ceux-ci sont constitués par la paroi extérieure 3 elle-même, dont la partie déployable/pliable 18, double, c'est-à dire chaque soufflet longitudinal 14, où se trouve les membranes 9c perméables au gaz de stérilisation, est interposée entre deux parties collatérales 19a et 19b, étanches, formées par les parties des grandes faces 13 a et 13b attenantes à chaque volet 15a et 15b, là où se trouvent les lignes de pliage latérales 16a et 16b.

Avec cette structure, lorsque la partie déployable/pliable 18, double, est pliée sur elle-même vers l'intérieur de l'emballage 1, les deux parties collatérales 19a et 19b sont situées en vis-à-vis et à proximité l'une de l'autre.

Elles sont alors aptes à être scellées l'une à l'autre à leur périphérie extérieure 20, de façon étanche, autour de la zone 9c perméable au gaz de stérilisation en forme de membrane 9c. Avec une telle structure, dans ce cas, les deux parties collatérales 19a et 19b empêchent le gaz de stérilisation d'accéder à la zone 9c perméable au gaz de stérilisation en forme de membrane 9c.

Le procédé de mise en oeuvre d'un tel emballage 1 est tel que l'on dispose d'un tel emballage 1, avec une membrane 9c perméable au gaz de stérilisation formée par une partie de la paroi extérieure 3, vide du contenu biopharmaceutique à stériliser C_{às}, et dont l'ouverture d'introduction 6, située à une extrémité, est ouverte. On dispose de moyens d'ouverture/fermeture 12, ici la paroi extérieure 3 elle-même et plus précisément encore les deux parties collatérales 19a et 19b. On dispose également d'une source de gaz de stérilisation. On dispose enfin du contenu biopharmaceutique à stériliser C_{às}.

Via l'ouverture d'introduction 6, on introduit le contenu biopharmaceutique à stériliser C_{às} directement dans l'espace intérieur de réception 2.

Puis, on ferme l'ouverture d'introduction 6 de façon étanche, en scellant le bord transversal 5 jusqu'ici ouvert.

Puis, alors que le passage d'amenée 7 est ouvert, pour la raison que la paroi extérieure 3 est déployée, de sorte que les moyens d'ouverture/fermeture 12, ici les deux parties collatérales 19a et 19b, sont inactifs au regard de la fonction de bouchage, on amène le gaz de stérilisation depuis la source de gaz de stérilisation jusque dans l'espace intérieur 2 où se trouve le contenu biopharmaceutique à stériliser C_{às}. C'est ainsi que l'on stérilise le contenu biopharmaceutique.

De la même façon que précédemment, le gaz de stérilisation peut alors être retiré de l'espace intérieur 2 en passant au travers du passage 7 d'amenée de gaz de stérilisation qui constitue alors un passage d'évacuation du gaz de stérilisation vers l'extérieur.

De contenu biopharmaceutique à stériliser C_{às}, il devient contenu biopharmaceutique stérilisé Cₛ.

Une fois la stérilisation faite, on plie la partie déployable/pliable 18, c'est-à-dire les soufflets 14, sur elle-même vers l'intérieur de l'emballage 1.

Par suite, les deux parties collatérales 19a et 19b de chaque soufflet 14 sont situées en vis-à-vis et à proximité l'une de l'autre. On peut alors les sceller l'une à l'autre à leur périphérie extérieure 20, de façon étanche, de sorte à fermer le passage 7 d'amenée de gaz de stérilisation.

On a alors formé un emballage biopharmaceutique 1 stérilisé enfermant le contenu biopharmaceutique stérilisé Cₛ.

L'emballage biopharmaceutique 1 stérilisé est constitué de la paroi extérieure 3, le passage 7 d'amenée de gaz de stérilisation et les moyens d'ouverture/fermeture 12, en l'espèce les deux parties collatérales 19a et 19b, qui restent solidaires entre eux et enferment le contenu biopharmaceutique Cₛ stérilisé.

Avec cette disposition, et comme précédemment, l'emballage biopharmaceutique 1 stérilisé et le contenu biopharmaceutique Cₛ stérilisé peuvent subir un ou plusieurs tests d'intégrité à tout moment souhaité après la stérilisation.

On se réfère maintenant plus spécialement aux figures 5A et 5B.

Dans cette variante, la paroi extérieure 3 comprend une partie latérale 21 de forme cylindrique, où est ménagée l'ouverture d'introduction 6, et deux faces transversales d'extrémité 22a et 22b, ici circulaire, à l'état déployé, comme il est représenté sur la figure 5B.

Chacune des deux faces transversales d'extrémité 22a et 22b peut être pliée de le long d'une ligne de pliage diamétrale 23 vers l'intérieur de l'emballage 1, à l'état plié, comme il est représenté sur la figure 5B, de sorte que les deux demi-faces transversales d'extrémité 24a et 24b de chaque face transversale d'extrémité 22a ou 22b en résultant, sont alors situés en regard de parties 25a et 25b de la partie latérale cylindrique 21.

La partie déployable/pliable 18, c'est-à dire ici les deux faces transversales d'extrémité 22a et 22b formant la membrane 9d, est apte à être soit déployée de sorte que le passage 7 d'amenée de gaz de stérilisation est ouvert soit pliée sur elle-même vers l'intérieur de l'emballage 1 par suite des deux demi-faces transversales d'extrémité 24a et 24b avec la ligne de pliage diamétrale 23, en sorte que le passage 7 d'amenée de gaz de stérilisation soit alors fermé.

Lorsque les deux demies faces transversales d'extrémité 24a et 24b de chacune des faces transversales d'extrémité 22a et 22b sont repliées l'une sur l'autre vers l'intérieur de l'emballage 1, elles sont recouvertes par les deux parties 25a et 25b diamétralement opposées et attenantes de la partie latérale 21, lesquelles sont situées en vis-à-vis et à proximité l'une de l'autre.

Les deux parties 25a et 25b diamétralement opposées sont alors aptes à être scellées l'une à l'autre à leur périphérie extérieure, de façon étanche, autour de la zone 9d perméable au gaz de stérilisation en forme de membrane 9d. Avec une telle structure, dans ce cas, les deux parties 25a et 25b empêchent le gaz de stérilisation d'accéder à la zone 9d perméable au gaz de stérilisation en forme de membrane 9d.

Le procédé de mise en oeuvre d'un tel emballage 1 est analogue à celui décrit précédemment aux adaptions résultant de la structure de l'emballage 1 près. Ainsi, une fois la stérilisation faite, on plie la partie déployable/pliable 18, c'est-à-dire les deux demies faces transversales d'extrémité 24a et 24b de chacune des faces transversales d'extrémité 22a et 22b et on scelle les deux parties 25a et 25b autour de chaque membrane 9d. On a alors formé un emballage biopharmaceutique 1 stérilisé enfermant le contenu biopharmaceutique stérilisé Cₛ.

Comme précédemment, l'emballage biopharmaceutique 1 stérilisé est constitué de la paroi extérieure 3, le passage 7 d'amenée de gaz de stérilisation et les moyens d'ouverture/fermeture 12, en l'espèce les deux parties diamétrales 25a et 25b, qui restent solidaires entre eux et enferment le contenu biopharmaceutique Cₛ stérilisé. Un tel emballage biopharmaceutique 1 stérilisé et le contenu biopharmaceutique Cₛ stérilisé peuvent subir un ou plusieurs tests d'intégrité à tout moment souhaité après la stérilisation.

On se réfère maintenant plus spécialement à la figure 6.

Cette variante diffère en ce que la paroi extérieure 3 comprend une partie latérale 26 de forme cylindrique à une extrémité et de forme aplatie à l'autre extrémité, lorsque l'ouverture d'introduction 6 est fermée.

La partie de forme cylindrique à une extrémité est analogue à l'une des faces transversales d'extrémité 22a ou 22b, avec par conséquent deux demi faces transversales d'extrémité 24a et 24b et une ligne de pliage diamétrale 23, formant la membrane 9e perméable au gaz de stérilisation.

La partie de forme aplatie est ouverte, originellement, et constitue l'ouverture d'introduction 6 qui peut être scellée sur elle-même.

Avec une telle structure, il est possible de positionner l'ouverture d'introduction 6 à une extrémité de l'emballage plutôt que sur la partie latérale 26, comme dans la variante des figures 5A et 5B.

On se réfère maintenant à la figure 7.

Dans cette réalisation, la paroi extérieure 3 présente une forme générale de gaine, comme dans la réalisation de la figure 1.

Le passage 7 d'amenée de gaz de stérilisation n'inclut pas une membrane perméable au gaz de stérilisation, telle que les membranes 9a, 9b, 9c, 9d ou 9e précédemment décrites, mais un (ou le cas échéant plusieurs) tronçon de tube 27 d'amenée de gaz de stérilisation.

Un tel tronçon de tube 27 est situé à l'extérieur de l'emballage 1. Il forme vers l'amont l'orifice d'amenée amont 8 de gaz de stérilisation et vers l'aval l'orifice de distribution aval 9 de gaz de stérilisation. Cet orifice 9 est ménagé dans la paroi extérieure 3. Le tronçon de tube 27 d'amenée de gaz de stérilisation est solidaire de la paroi extérieure 3 de façon étanche.

Un (ou le cas échéant plusieurs) moyen 28 apte à retenir d'éventuels germes pathogènes, tel qu'une cartouche filtrante, est interposé sur le tronçon de tube 27 entre l'orifice d'amenée amont 8 et l'orifice de distribution aval 9.

Avec cette seconde réalisation, les moyens de commande 12 de la distribution de gaz de stérilisation sont, ici aussi, des moyens d'ouverture/fermeture 12 du passage d'amenée 7, à savoir un moyen d'arrêt 12c de flux, monté mobile ou amovible sur le tronçon de tube 27.

Un tel moyen d'arrêt 12c est par exemple une pince de fermeture ou un soudage transversal.

Le procédé de mise en oeuvre d'un tel emballage 1 dérive du procédé précédemment décrit en relation avec les autres réalisations.

On dispose d'un emballage biopharmaceutique 1 avec un tronçon de tube 27, de moyens d'ouverture/d'obturation 12 du passage d'amenée 7 de gaz de stérilisation ici un moyen d'arrêt de flux 12c, et du contenu biopharmaceutique à stériliser C_{às}.

Après que le contenu biopharmaceutique a été stérilisé, on interpose le moyen d'arrêt de flux 12c sur le tronçon de tube 27 de sorte à fermer le passage 7 d'amenée de gaz de stérilisation.

Comme précédemment, l'emballage biopharmaceutique 1 stérilisé est constitué de ses parties constitutives solidaires entre elles, sans nécessité de devoir être sectionné, de sorte que l'emballage biopharmaceutique 1 stérilisé et le contenu biopharmaceutique stérilisé Cₛ peuvent subir un ou plusieurs tests d'intégrité à tout moment souhaité après la stérilisation.

Comme précédemment, par conséquent, le procédé est tel que l'on garde solidaires les parties constitutives de l'emballage stérilisé, sans devoir procéder à une section en plusieurs parties.

Dans toutes les réalisations représentées, l'espace intérieur 2 s'étend sur substantiellement toute la longueur de la paroi extérieure 3. De plus, à l'exception de la réalisation des figures 5A et 5B, l'ouverture d'introduction 7 est située à une extrémité de la paroi extérieure 3. D'autres formes de réalisation peuvent toutefois être envisagées. D'autre part, l'orifice d'amenée amont 8 de gaz et l'ouverture d'introduction 6 du contenu biopharmaceutique à stériliser C_{às} à stériliser sont non seulement distincts mais écartés l'un de l'autre.

Dans les réalisations des figures 1, 2, 3, 5A, 5B, il est également prévu que l'emballage 1 comporte un (ou plusieurs) embout 29 d'entrée/sortie de gaz.

Un tel embout 29 est monté sur la paroi extérieure 3. Il est en communication avec l'espace intérieur 2. Il est apte à être amené à l'état ouvert ou à l'état fermé par actionnement de moyens d'obturation adaptés, tels que des moyens de bouchage. Il est apte à être associé en communication avec une source ou une sortie de gaz. Ainsi, cet embout 29 permet de faire entrer dans ou de faire sortir de l'espace intérieur 2, un gaz fonctionnel, ou de créer dans l'espace intérieur 2, une pression ou une dépression.

Un tel gaz peut être par exemple celui mis en oeuvre pour un procédé de contrôle d'intégrité, afin d'avoir un différentiel de pression entre l'espace intérieur 2 et l'ambiance extérieure de l'emballage 1.

Le procédé de mise en oeuvre de l'emballage 1 est tel qu'une fois la stérilisation faite, on ne sectionne pas l'emballage biopharmaceutique 1 en plusieurs tronçons, ce qui permet d'éviter les inconviénients d'un tel sectionnement : temps passé, moyens techniques de sectionnement, risque de détérioration de la paroi extérieure 3.

L'invention vise non seulement l'emballage 1 vide avant stérilisation de l'intérieur et de son contenu C_{às}, mais également l'emballage stérilisé 1 enfermant un contenu biopharmaceutique stérilisé Cₛ, comme il a été obtenu par la mise en oeuvre du procédé selon l'une des revendications 11 à 15.

Un tel emballage stérilisé 1 empli de son contenu biopharmaceutique stérilisé C_{às} comporte également une ouverture d'extraction depuis l'intérieur vers l'extérieur de l'emballage du contenu biopharmaceutique stérilisé C_{às}.

Cette ouverture d'extraction est fermée de façon étanche pendant la stérilisation. Elle est apte par la suite à être ouverte en sorte d'extraire le contenu biopharmaceutique de l'espace intérieur.

Une telle ouverture d'extraction peut être simplement une déchirure ou une coupe de la paroi extérieure 3.

Comme indiqué, l'emballage biopharmaceutique 1 stérilisé et le contenu biopharmaceutique Cₛ stérilisé peuvent subir un ou plusieurs tests d'intégrité à tout moment souhaité après la stérilisation.

La description vise donc également le procédé qui comporte toutes les étapes précédemment décrites pour réaliser l'emballage biopharmaceutique 1 stérilisé avec son contenu biopharmaceutique Cₛ stérilisé et dans lequel, à tout moment souhaité après la stérilisation, on fait subir à cet emballage biopharmaceutique stérilisé enfermant le contenu biopharmaceutique stérilisé Cₛ un ou plusieurs tests d'intégrité du type requérant une étanchéité au gaz de l'emballage.

Selon une autre caractéristique, l'un ou l'autre des modes de réalisation de l'emballage biopharmaceutique 1 tels que précédemment décrits peut également intégrer une ouverture délimitée par une bride annulaire sur laquelle est montée un moyen mobile ou amovible de transfert aseptique, correspondant par exemple aux portes qui sont utilisées dans les dispositifs de transfert aseptique de type à double porte.

A titre exemplatif et nullement limitatif, on peut citer les dispositifs connus sous la maque BIOSAFE® et les dispositifs de jonction étanche entre une première enceinte et une seconde enceinte isolées d'un milieu extérieur décrits dans le document EP-A-0688020.

En outre, l'on pourrait également utiliser des dispositifs de jonction étanche similaires à ceux connus sous la maque BIOSAFE® mais permettant de réaliser une pluralité d'ouvertures successives du moyen mobile ou amovible de transfert asceptique.

Selon différentes variantes de réalisation, ce moyen mobile ou amovible de transfert aseptique peut soit coopérer avec l'ouverture d'introduction 6 pour assurer la fermeture de cette ouverture d'introduction 6 de façon étanche soit coopérer avec une portion de la paroi extérieure 3 distincte de la membrane 9 perméable au gaz de stérilisation de manière à former une ouverture secondaire apte à être ouverte puis fermée de façon étanche.

Il est ainsi possible de transférer le contenu biopharmaceutique stérilisé enfermé à l'intérieur de l'emballage biopharmaceutique 1 au travers du moyen mobile ou amovible de transfert aseptique et vers une enceinte isolée sans risquer que ledit contenu biopharmaceutique stérilisé ne soit mis au contact du milieu extérieur et contaminé par lui.

Une telle réalisation permet ainsi de diminuer également les risques de contamination du contenu biopharmaceutique après que ce dernier ait été stérilisé.

## Revendications

1. Emballage biopharmaceutique (1) stérilisable, apte à enfermer un contenu biopharmaceutique à stériliser, comprenant :
▪ une paroi extérieure (3) souple, étanche aux gaz et aux germes pathogènes,
▪ une ouverture d'introduction (6) depuis l'extérieur vers l'intérieur de l'emballage (1) du contenu biopharmaceutique à stériliser, apte par la suite à être fermée de façon étanche,
▪ un espace intérieur (2) apte à recevoir le contenu biopharmaceutique,
▪ un passage d'amenée (7) de gaz de stérilisation depuis l'extérieur de l'emballage (1), comprenant au moins un orifice d'amenée amont (8) apte à être associé en communication (10) avec une source de gaz de stérilisation, au moins un orifice de distribution aval (9) de gaz de stérilisation débouchant dans l'espace intérieur (2), et une communication (10) entre l'orifice d'amenée amont (8) et l'orifice de distribution aval (9),
▪ l'ouverture d'introduction (6) du contenu biopharmaceutique à stériliser est distincte du passage d'amenée (7) de gaz de stérilisation, de sorte que l'ouverture d'introduction (6) du contenu biopharmaceutique à stériliser peut être fermée indépendamment et avant la fermeture du passage d'amenée (7) de gaz de stérilisation,
▪ l'ouverture d'introduction (6) du contenu biopharmaceutique à stériliser est directement adjacente à l'espace intérieur (2) de réception du contenu biopharmaceutique à stériliser et du contenu biopharmaceutique stérilisé, de sorte que le contenu biopharmaceutique à stériliser est introduit directement dans l'espace intérieur (2) depuis l'extérieur de l'emballage (1),
▪ les moyens (12) de commande de la distribution de gaz de stérilisation dans l'espace intérieur (2) sont des moyens (12) d'ouverture/fermeture du passage d'amenée (7) de gaz de stérilisation, de sorte que le passage d'amenée (7) de gaz de stérilisation peut être ouvert ou fermé indépendamment de l'ouverture ou fermeture de l'ouverture d'introduction (6),
**caractérisé par le fait que** :
▪ le passage d'amenée (7) inclut au moins une membrane (9) perméable au gaz de stérilisation, surfaciquement étendue, formant un orifice de distribution aval (9) de gaz de stérilisation distribué sur la surface de la membrane (9), la membrane (9) formant une partie de l'enveloppe de l'espace intérieur (2) et constituant un moyen de filtration apte à retenir d'éventuels germes pathogènes,
▪ ladite membrane (9) perméable au gaz de stérilisation est constituée par une zone perméable de la paroi extérieure (3) de l'emballage (1) appartenant à une partie déployable/pliable de la paroi extérieure (3) de l'emballage (1) et formant le passage d'amenée (7) de gaz de stérilisation, cette partie déployable/pliable étant apte à être soit déployée de sorte que le passage d'amenée (7) de gaz de stérilisation est ouvert soit pliée sur elle-même vers l'intérieur de l'emballage (1) en sorte que le passage d'amenée (7) de gaz de stérilisation soit fermé,
▪ ledit emballage est configuré pour qu'une fois la stérilisation faite, la paroi extérieure (3), le passage d'amenée (7) de gaz de stérilisation et les moyens (12) d'ouverture/fermeture restent solidaires entre eux de sorte à former un

2. Emballage biopharmaceutique (1) stérilisable selon la revendication 1, dans lequel la partie de la paroi extérieure (3) de l'emballage (1) apte à être soit à l'état déployé soit à l'état plié sur elle-même vers l'intérieur est un soufflet longitudinal (14) de la paroi extérieure (3).

3. Emballage biopharmaceutique (1) stérilisable selon la revendication 1, dans lequel la partie de la paroi extérieure (3) de l'emballage (1) apte à être soit à l'état déployé soit à l'état plié sur elle-même vers l'intérieur est une partie transversale d'extrémité (22a, 22b) de la paroi extérieure (3).

4. Emballage biopharmaceutique (1) stérilisable selon l'une quelconque des revendications 1 à 3, dans lequel les moyens (12) d'ouverture/fermeture sont constitués par la paroi extérieure (3) elle-même, dont la partie déployable/pliable où se trouve la zone perméable au gaz de stérilisation, est interposée entre deux parties collatérales étanches (19a, 19b) situées de part et d'autre de la partie déployable/pliable, de manière telle que lorsque la partie déployable/pliable est pliée sur elle-même vers l'intérieur de l'emballage (1), les deux parties collatérales (19a, 19b), alors situées en vis-à-vis et à proximité l'une de l'autre, sont aptes à être scellées l'une à l'autre à leur périphérie extérieure (20) de façon étanche, autour de la zone perméable au gaz de stérilisation, de sorte à empêcher le gaz de stérilisation d'accéder à zone perméable.

5. Emballage biopharmaceutique (1) stérilisable selon l'une quelconque des revendications 1 à 4, dans lequel l'espace intérieur (2) s'étend sur toute la longueur de la paroi extérieure (3) en forme de gaine, et dans lequel l'ouverture d'introduction (6) du contenu biopharmaceutique à stériliser est située à une extrémité de la paroi extérieure (3) en forme de gaine.

6. Emballage biopharmaceutique (1) stérilisable selon l'une quelconque des revendications 1 à 5, dans lequel le au moins un orifice d'amenée amont (8) de gaz et l'ouverture d'introduction (6) du contenu biopharmaceutique à stériliser sont écartés l'un de l'autre.

7. Emballage biopharmaceutique (1) stérilisable selon l'une quelconque des revendications 1 à 6, qui comporte également au moins un embout d'entrée/sortie de gaz, monté sur la paroi extérieure (3), en communication (10) avec l'espace intérieur (2), apte à être amené à l'état ouvert ou à l'état fermé par actionnement de moyens d'obturation, de sorte à permettre de faire entrer dans ou de faire sortir de l'espace intérieur (2), un gaz fonctionnel.

8. Emballage biopharmaceutique (1) stérilisable selon l'une quelconque des revendications 1 à 7, comportant également une ouverture délimitée par une bride annulaire sur laquelle est monté un moyen mobile ou amovible de transfert aseptique, tel qu'une porte mobile ou amovible de transfert aseptique de type à double porte.

9. Emballage biopharmaceutique (1) stérilisable selon la revendication 8, dans lequel le moyen mobile ou amovible de transfert aseptique coopère avec l'ouverture d'introduction (6) et est apte à assurer la fermeture de cette ouverture d'introduction (6) de façon étanche.

10. Emballage biopharmaceutique (1) stérilisable selon la revendication 8, dans lequel le moyen mobile ou amovible de transfert aseptique coopère avec une portion de la paroi extérieure (3) distincte de la membrane (9) perméable au gaz de stérilisation de manière à former une ouverture secondaire apte à être ouverte puis fermée de façon étanche.

11. Procédé de mise en oeuvre d'un emballage biopharmaceutique (1) stérilisable selon l'une quelconque des revendications 1 à 10, en vu d'y enfermer et d'y stériliser un contenu biopharmaceutique, dans lequel :
▪ on dispose d'un tel emballage (1), vide du contenu biopharmaceutique, et dont l'ouverture d'introduction (6) est ouverte,
▪ on dispose du contenu biopharmaceutique à stériliser,
▪ on dispose d'une source de gaz de stérilisation,
▪ via l'ouverture d'introduction (6), on introduit le contenu biopharmaceutique à stériliser directement dans l'espace intérieur (2) de réception,
▪ puis, on ferme l'ouverture d'introduction (6) de façon étanche,
▪ puis, alors que le passage d'amenée (7) de gaz de stérilisation est ouvert, on amène le gaz de stérilisation depuis la source de gaz de stérilisation jusque dans l'espace intérieur (2) de réception où se trouve le contenu biopharmaceutique à stériliser, de sorte à stériliser le contenu biopharmaceutique,
▪ puis, on met en oeuvre les moyens (12) d'ouverture/fermeture de sorte à fermer le passage d'amenée (7) de gaz de stérilisation; dite membrane (9) perméable au gaz de stérilisation est constituée par la zone perméable de la paroi extérieure (3) de l'emballage (1) appartenant à la partie déployable/pliable de la paroi extérieure (3) de l'emballage (1) et formant le passage d'amenée (7) de gaz de stérilisation, cette partie déployable/pliable étant apte à être soit déployée de sorte que le passage d'amenée (7) de gaz de stérilisation est ouvert soit pliée sur elle-même vers l'intérieur de l'emballage (1) en sorte que le passage d'amenée (7) de gaz de stérilisation soit fermé,
▪ et on forme ainsi un emballage biopharmaceutique (1) stérilisé enfermant le contenu biopharmaceutique stérilisé pouvant subir un ou plusieurs tests d'intégrité du type avec pression différentielle requérant une étanchéité au gaz de l'emballage, à tout moment souhaité après la stérilisation.

12. Procédé selon la revendication 11, dans lequel :
▪ on dispose d'un emballage biopharmaceutique (1) selon la revendication 1, avec une membrane (9) perméable au gaz de stérilisation constituée par une zone perméable de la paroi extérieure (3) de l'emballage (1) appartenant à une partie déployable/pliable, et on dispose de moyens (12) d'ouverture/fermeture constitués par la paroi extérieure (3) dont la partie déployable/pliable est interposée entre deux parties collatérales étanches,
▪ et, après que le contenu biopharmaceutique a été stérilisé, on plie la partie déployable/pliable sur elle-même vers l'intérieur de l'emballage (1), de sorte que les deux parties collatérales soient situées en vis-à-vis et à proximité l'une de l'autre, et on les scelle l'une à l'autre à leur périphérie extérieure de façon étanche, autour de la zone perméable au gaz de stérilisation, de sorte à fermer le passage d'amenée (7) de gaz de stérilisation.

13. Procédé selon l'une quelconque des revendications 11 à 12, dans lequel, une fois la stérilisation faite, on garde solidaires la paroi extérieure (3), le passage d'amenée (7) de gaz de stérilisation et les moyens (12) d'ouverture/fermeture de sorte à former l'emballage biopharmaceutique (1) stérilisé enfermant le contenu biopharmaceutique stérilisé.

14. Procédé selon la revendication 13, dans lequel, une fois la stérilisation faite, on ne sectionne pas l'emballage biopharmaceutique (1) en plusieurs tronçons.

15. Procédé selon l'une quelconque des revendications 11 à 14, dans lequel, à tout moment souhaité après la stérilisation, on fait subir à l'emballage biopharmaceutique (1) stérilisé enfermant le contenu biopharmaceutique stérilisé un ou plusieurs tests d'intégrité du type requérant une étanchéité au gaz de l'emballage (1).

16. Emballage biopharmaceutique (1) stérilisé enfermant un contenu biopharmaceutique stérilisé **caractérisé par le fait qu'**il est obtenu par la mise en oeuvre du procédé selon l'une quelconque des revendications 11 à 15, l'emballage (1) comportant également une ouverture d'extraction depuis l'intérieur vers l'extérieur de l'emballage (1) le contenu biopharmaceutique stérilisé, fermée de façon étanche et apte par la suite à être ouverte en sorte d'extraire le contenu biopharmaceutique de l'espace intérieur (2).

## Patentansprüche

1. Sterilisierbare biopharmazeutische Verpackung (1), welche dazu geeignet ist, einen zu sterilisierenden biopharmazeutischen Inhalt einzuschließen, umfassend:
▪ eine flexible, gasdichte und gegenüber pathogenen Keimen dichte Außenwand (3),
▪ eine Einführöffnung (6) von außen in das Innere der Verpackung (1) zum Einführen des zu sterilisierenden biopharmazeutischen Inhalts, welche nachfolgend dicht verschlossen werden kann,
▪ einen Innenraum (2), welcher dazu geeignet ist, den biopharmazeutischen Inhalt aufzunehmen,
▪ einen Zuführdurchlass (7) für Sterilisationsgas vom Äußeren der Verpackung (1), welcher wenigstens eine stromaufwärtige Zuführöffnung (8) umfasst, die dazu geeignet ist, in kommunizierender Verbindung (10) mit einer Sterilisationsgasquelle zu stehen, wenigstens eine in den Innenraum (2) mündende stromabwärtige Verteilungsöffnung (9) für Sterilisationsgas und eine Verbindung (10) zwischen der stromaufwärtigen Zuführöffnung (8) und der stromabwärtigen Verteilungsöffnung (9),
▪ wobei die Einführöffnung (6) für den zu sterilisierenden biopharmazeutischen Inhalt von dem Zuführdurchlass (7) für Sterilisationsgas verschieden ist, derart dass die Einführöffnung (6) für den zu sterilisierenden biopharmazeutischen Inhalt unabhängig und vor dem Verschließen des Zuführdurchlasses (7) für Sterilisationsgas verschlossen werden kann,
▪ wobei die Einführöffnung (6) für den zu sterilisierenden biopharmazeutischen Inhalt direkt benachbart zum Innenraum (2) ist, für die Aufnahme des zu sterilisierenden biopharmazeutischen Inhalts und des sterilisierten biopharmazeutischen Inhalts, derart dass der zu sterilisierende pharmazeutische Inhalt vom Äußeren der Verpackung (1) direkt in den Innenraum (2) eingeführt werden kann,
▪ wobei die Mittel (12) zur Steuerung der Verteilung des Sterilisationsgases im Innenraum (2) Mittel (12) zum Öffnen/Verschließen des Zuführdurchlasses (7) für Sterilisationsgas sind, derart, dass der Zuführdurchlass (7) für Sterilisationsgas unabhängig vom Öffnen oder Verschließen der Einführöffnung (6) geöffnet oder verschlossen werden kann,
**dadurch gekennzeichnet, dass**:
▪ der Zuführdurchlass (7) für Sterilisationsgas wenigstens eine für das Sterilisationsgas durchlässige Membran (9) umfasst, die sich flächenmäßig erstreckt und eine stromabwärtige Verteilungsöffnung (9) für an der Oberfläche der Membran (9) verteiltes Sterilisationsgas bildet, wobei die Membran (9) einen Teil der Hülle des Innenraums (2) bildet und ein Filtrationsmittel bildet, welches dazu geeignet ist, mögliche pathogene Keime zurückzuhalten,
▪ die für das Sterilisationsgas durchlässige Membran (9) durch einen durchlässigen Bereich der Außenwand (3) der Verpackung (1) gebildet ist, zugehörig zu einem entfaltbaren/faltbaren Teil der Außenwand (3) der Verpackung (1), und welche den Zuführdurchlass (7) für Sterilisationsgas bildet, wobei dieser entfaltbare/faltbare Teil dazu geeignet ist, entweder derart entfaltet zu werden, dass der Zuführdurchlass (7) für Sterilisationsgas geöffnet ist, oder derart auf sich selbst zum Inneren der Verpackung (1) hin gefaltet zu werden, dass der Zuführdurchlass (7) für Sterilisationsgas verschlossen ist,
▪ die Verpackung derart ausgebildet ist, dass nach erfolgter Sterilisation die Außenwand (3), der Zuführdurchlass (7) für Sterilisationsgas und die Mittel (12) zum Öffnen/Verschließen derart miteinander verbunden bleiben, dass sie eine sterilisierte biopharmazeutische Verpackung (1) bilden, welche den sterilisierten biopharmazeutischen Inhalt einschließt und nach der Sterilisation zu jedem gewünschten Zeitpunkt einem oder mehreren Integritätstests vom Typ Differenzialdruck unterzogen werden kann, bei welchem eine Gasabdichtung der Verpackung erforderlich ist.

2. Sterilisierbare biopharmazeutische Verpackung (1) nach Anspruch 1, bei welcher der Teil der Außenwand (3) der Verpackung (1), der dazu geeignet ist, entweder entfaltet oder in dem auf sich zum Inneren hin gefalteten Zustand zu sein, ein Faltenbalg in Längsrichtung (14) der Außenwand (3) ist.

3. Sterilisierbare biopharmazeutische Verpackung (1) nach Anspruch 1, bei welcher der Teil der Außenwand (3) der Verpackung (1), der dazu geeignet ist, entweder entfaltet oder in dem auf sich zum Inneren hin gefalteten Zustand zu sein, ein querverlaufender Endteil (22a, 22b) der Außenwand (3) ist.

4. Sterilisierbare biopharmazeutische Verpackung (1) nach einem der Ansprüche 1 bis 3, bei welcher die Mittel (12) zum Öffnen/Verschließen durch die Außenwand (3) selbst gebildet sind, wobei der entfaltbare/faltbare Teil, in dem sich der für das Sterilisationsgas durchlässige Bereich befindet, zwischen zwei dichten Seitenteilen (19a, 19b) eingefügt ist, die beidseits des entfaltbaren/faltbare Teils angeordnet sind, so dass, wenn der entfaltbare/faltbare Teil auf sich selbst zum Inneren der Verpackung (1) hin gefaltet wird, die beiden dann gegenüber und in Nachbarschaft zueinander angeordneten Seitenteile (19a, 19b) dazu geeignet sind, aneinander an ihrem Außenumfang (20) rund um den für das Sterilisationsgas durchlässigen Bereich dicht versiegelt zu werden, so dass das Sterilisationsgas nicht in den durchlässigen Bereich gelangen kann.

5. Sterilisierbare biopharmazeutische Verpackung (1) nach einem der Ansprüche 1 bis 4, bei welcher sich der Innenraum (2) über im Wesentlichen die gesamte Länge der Außenwand (3) in Form einer Hülle erstreckt, und wobei sich die Einführöffnung (6) für den zu sterilisierenden biopharmazeutischen Inhalt an einem Ende der Außenwand (3) in Form einer Hülle befindet.

6. Sterilisierbare biopharmazeutische Verpackung (1) nach einem der Ansprüche 1 bis 5, bei welcher die wenigstens eine stromaufwärtige Zuführöffnung (8) für Gas und die Einführöffnung (6) für den zu sterilisierenden biopharmazeutischen Inhalt zueinander beabstandet sind.

7. Sterilisierbare biopharmazeutische Verpackung (1) nach einem der Ansprüche 1 bis 6, welche ferner wenigstens einen Gas-Eingangs-/Ausgansstutzen umfasst, der an der Außenwand (3) angebracht ist und in Verbindung (10) mit dem Innenraum (2) steht, dazu geeignet, durch Betätigung von Verschlussmitteln in den geöffneten Zustand oder verschlossenen Zustand gebracht zu werden, um zu ermöglichen, dass ein Funktionsgas in den Innenraum (2) hinein oder aus ihm heraus gelangt.

8. Sterilisierbare biopharmazeutische Verpackung (1) nach einem der Ansprüche 1 bis 7, welche ferner begrenzt ist durch einen ringförmigen Flansch, woran ein bewegliches oder entfernbares Mittel für einen aseptischen Transfer befestigt ist, wie beispielsweise eine bewegliche oder entfernbare Klappe für einen aseptischen Transfer vom Typ Doppelklappe.

9. Sterilisierbare biopharmazeutische Verpackung (1) nach Anspruch 8, bei welcher das bewegliche oder entfernbare Mittel für den aseptischen Transfer mit der Einführöffnung (6) zusammenwirkt und dazu geeignet ist, das dichte Verschließen dieser Einführöffnung (6) sicherzustellen.

10. Sterilisierbare biopharmazeutische Verpackung (1) nach Anspruch 8, bei welcher das bewegliche oder entfernbare Mittel für den aseptischen Transfer mit einem von der für das Sterilisationsgas durchlässigen Membran (9) verschiedenen Teil der Außenwand (3) derart zusammenwirkt, dass eine Sekundäröffnung gebildet wird, die geöffnet und nachfolgend auf dichte Weise verschlossen werden kann.

11. Verfahren zur Herstellung einer sterilisierbaren biopharmazeutischen Verpackung (1) nach einem der Ansprüche 1 bis 10, mit dem Ziel, einen biopharmazeutischen Inhalt einzuschließen und zu sterilisieren, wobei:
▪ eine solche Verpackung (1) ohne biopharmazeutischen Inhalt vorhanden ist, deren Einführöffnung (6) geöffnet ist,
▪ der zu sterilisierende biopharmazeutische Inhalt vorhanden ist,
▪ eine Sterilisationsgasquelle vorhanden ist,
▪ durch die Einführöffnung (6) der zu sterilisierende biopharmazeutische Inhalt direkt in den Aufnahmeinnenraum (2) eingeführt wird,
▪ nachfolgend die Einführöffnung (6) dicht verschlossen wird,
▪ nachfolgend, wenn der Zuführdurchlass (7) für Sterilisationsgas geöffnet ist, das Sterilisationsgas von der Sterilisationsgasquelle in den Aufnahmeinnenraum (2) geführt wird, wo sich der zu sterilisierende biopharmazeutische Inhalt befindet, um den biopharmazeutischen Inhalt zu sterilisieren,
▪ nachfolgend die Mittel (12) zum Öffnen/Verschließen betätigt werden, um den Zuführdurchlass (7) für Sterilisationsgas zu verschließen, wobei die für das Sterilisationsgas durchlässige Membran (9) durch einen durchlässigen Bereich der Außenwand (3) der Verpackung (1) gebildet ist, zugehörig zu einem entfaltbaren/faltbaren Teil der Außenwand (3) der Verpackung (1), und welche den Zuführdurchlass (7) für Sterilisationsgas bildet, wobei der entfaltbare/faltbare Teil dazu geeignet ist, entweder derart entfaltet zu werden, dass der Zuführdurchlass (7) für Sterilisationsgas geöffnet ist, oder derart auf sich selbst zum Inneren der Verpackung (1) hin gefaltet zu werden, dass der Zuführdurchlass (7) für Sterilisationsgas verschlossen ist,
▪ derart eine sterilisierte biopharmazeutische Verpackung (1) gebildet wird, die den sterilisierten biopharmazeutischen Inhalt einschließt und zu jedem gewünschten Zeitpunkt einem oder mehreren Integritätstests vom Typ Differenzialdruck unterzogen werden kann, bei welchem eine Gasabdichtung der Verpackung erforderlich ist.

12. Verfahren nach Anspruch 11, wobei:
▪ eine biopharmazeutische Verpackung (1) nach Anspruch 1 vorhanden ist, mit einer für Sterilisationsgas durchlässigen Membran (9), die durch einen durchlässigen Bereich der Außenwand (3) der Verpackung (1) gebildet ist, zugehörig zu einem entfaltbaren/faltbaren Teil, wobei durch die Außenwand (3) gebildete Mittel (12) zum Öffnen/Verschließen vorliegen, deren entfaltbarer/faltbarer Teil zwischen zwei dichten Seitenteilen eingefügt ist,
▪ und wobei, nachdem der biopharmazeutische Inhalt sterilisiert wurde, der entfaltbare/faltbare Teil zum Inneren der Verpackung (1) hin derart gefaltet wird, dass beiden Seitenteile einander gegenüber und in Nachbarschaft angeordnet sind und sie aneinander an ihrem Außenumfang rund um den für das Sterilisationsgas durchlässigen Bereich dicht versiegelt werden, so dass der Zuführdurchlass (7) für Sterilisationsgas verschlossen wird.

13. Verfahren nach einem der Ansprüche 11 bis 12, bei welchem nach erfolgter Sterilisation die Außenwand (3), der Zuführdurchlass (7) für Sterilisationsgas und die Mittel (12) zum Öffnen/Verschließen verbunden bleiben, derart, dass die sterilisierte biopharmazeutische Verpackung (1) gebildet wird, die den sterilisierten biopharmazeutischen Inhalt einschließt.

14. Verfahren nach Anspruch 13, bei welchem nach erfolgter Sterilisation die biopharmazeutische Verpackung (1) nicht in mehrere Abschnitte unterteilt wird.

15. Verfahren nach einem der Ansprüche 11 bis 14, bei welchem zu jedem gewünschten Zeitpunkt nach der Sterilisation die sterilisierte biopharmazeutische Verpackung (1), die den sterilisierten biopharmazeutischen Inhalt einschließt, einem oder mehreren Integritätstests vom Typ Differenzialdruck, bei welchem eine Gasabdichtung der Verpackung (1) erforderlich ist, unterzogen wird.

16. Sterilisierte biopharmazeutische Verpackung (1), welche einen sterilisierten biopharmazeutischen Inhalt einschließt, **dadurch gekennzeichnet, dass** sie durch Ausführen des Verfahrens nach einem der Ansprüche 11 bis 15 erhalten wird, wobei die Verpackung (1) ferner eine Extraktionsöffnung vom Inneren zum Äußeren der Verpackung (1) aufweist, welche dicht verschlossen ist und nachfolgend geöffnet werden kann, um den biopharmazeutischen Inhalt aus dem Innenraum (2) zu extrahieren.

## Claims

1. Sterilizable biopharmaceutical packaging (1), capable of enclosing biopharmaceutical contents to be sterilized, comprising:
▪ An outer wall (3) that is flexible and sealed against gases and pathogenic germs,
▪ An opening (6) for introduction of the biopharmaceutical contents to be sterilized from the outside to the inside of the packaging (1), as a result capable of being closed in an airtight manner,
▪ An inside space (2) that is capable of receiving the biopharmaceutical contents,
▪ A sterilization gas conveyance channel (7) from the outside of the packaging (1), comprising at least one upstream opening (8) for intake capable of being associated in communication (10) with a sterilization gas source, at least one downstream opening (9) for distribution of sterilization gas emptying into the inside space (2), and a communication (10) between the upstream intake opening (8) and the downstream distribution opening (9),
▪ The opening (6) for introduction of the biopharmaceutical contents to be sterilized is separate from the sterilization gas conveyance channel (7), in such a way that the opening (6) for introduction of the biopharmaceutical contents to be sterilized can be closed independently and before the closing of the sterilization gas conveyance channel (7),
▪ The opening (6) for introduction of the biopharmaceutical contents to be sterilized is directly adjacent to the inside space (2) for receiving the biopharmaceutical contents to be sterilized and the sterilized biopharmaceutical contents, in such a way that the biopharmaceutical contents to be sterilized are introduced directly into the inside space (2) from the outside of the packaging (1),
▪ The means (12) for control of the sterilization gas distribution in the inside space (2) are opening/closing means (12) of the sterilization gas conveyance channel (7), in such a way that the sterilization gas conveyance channel (7) can be opened or closed independently of the opening or closing of the introduction opening (6),
**characterized in that**:
▪ The conveyance channel (7) includes at least one large-surface membrane (9) that is permeable to the sterilization gas, forming a downstream opening (9) for distribution of sterilization gas distributed over the surface of the membrane (9), the membrane (9) forming a part of the envelope of the inside space (2) and constituting a filtering means that is capable of stopping any pathogenic germs,
▪ Said membrane (9) that is permeable to sterilization gas consists of a permeable zone of the outer wall (3) of the packaging (1) that belongs to a deployable/foldable part of the outer wall (3) of the packaging (1) and that forms the sterilization gas conveyance channel (7), with this deployable/foldable part being capable of being either deployed in such a way that the sterilization gas conveyance channel (7) is open, or folded on itself toward the inside of the packaging (1) in such a way that the sterilization gas conveyance channel (7) is closed,
▪ Said packaging being configured so that once the sterilization is done, the outer wall (3), the sterilization gas conveyance channel (7), and the opening/closing means (12) remain integral with one another in such a way as to form a sterilized biopharmaceutical packaging (1) that encloses the sterilized biopharmaceutical contents that can undergo one or more integrity tests of the type with differential pressure requiring sealing of the packaging against gas at any time desired after sterilization.

2. Sterilizable biopharmaceutical packaging (1) according to Claim 1, wherein the part of the outer wall (3) of the packaging (1) that is capable of being either in the deployed state or in the state folded on itself toward the inside is a longitudinal gusset (14) of the outer wall (3).

3. Sterilizable biopharmaceutical packaging (1) according to Claim 1, wherein the part of the outer wall (3) of the packaging (1) that is capable of either being in the deployed state or in the state folded on itself toward the inside is a transverse end part (22a, 22b) of the outer wall (3).

4. Sterilizable biopharmaceutical packaging (1) according to any one of Claims 1 to 3, wherein the opening/closing means (12) consist of the outer wall (3) itself, whose deployable/foldable part where the zone that is permeable to sterilization gas is located is inserted between two airtight facing side parts (19a, 19b) located on both sides of the deployable/foldable part, in such a way that when the deployable/foldable part is folded on itself toward the inside of the packaging (1), the two facing side parts (19a, 19b), then located opposite and close together, are capable of being sealed to one another on their outer periphery (20) in an airtight manner, around the zone that is permeable to the sterilization gas, in such a way as to prevent the sterilization gas from accessing the permeable zone.

5. Sterilizable biopharmaceutical packaging (1) according to any one of Claims 1 to 4, wherein the inside space (2) extends over the entire length of the outer wall (3) in sheath form, and in which the introduction opening (6) of the biopharmaceutical contents to be sterilized is located at one end of the outer wall (3) in sheath form.

6. Sterilizable biopharmaceutical packaging (1) according to any one of Claims 1 to 5, wherein the at least one upstream opening (8) for intake of gas and the introduction opening (6) of the biopharmaceutical contents to be sterilized are separated from one another.

7. Sterilizable biopharmaceutical packaging (1) according to any one of Claims 1 to 6, which also comprises at least one gas inlet/outlet end fitting, mounted on the outer wall (3), in communication (10) with the inside space (2), able to be brought into the open state or into the closed state by actuating closing means, in such a way as to make it possible to enable a functional gas to enter into or exit from the inside space (2).

8. Sterilizable biopharmaceutical packaging (1) according to any one of Claims 1 to 7, also comprising an opening that is delimited by an annular flange on which is mounted a movable or detachable means of aseptic transfer, such as a movable or detachable door for aseptic transfer of the double-door type.

9. Sterilizable biopharmaceutical packaging (1) according to Claim 8, wherein the movable or detachable means for aseptic transfer works with the introduction opening (6) and is capable of ensuring the closing of this introduction opening (6) in an airtight manner.

10. Sterilizable biopharmaceutical packaging (1) according to Claim 8, wherein the movable or detachable means for aseptic transfer works with a portion of the outer wall (3) that is separate from the membrane (9) that is permeable to sterilization gas in such a way as to form a secondary opening that is capable of being opened and then closed in an airtight manner.

11. Process for implementing a sterilizable biopharmaceutical packaging (1) according to any one of Claims 1 to 10, for the purpose of enclosing and sterilizing biopharmaceutical contents therein, wherein:
▪ Such a packaging (1), empty of biopharmaceutical contents, and whose introduction opening (6) is open, is used,
▪ The biopharmaceutical contents to be sterilized are used,
▪ A sterilization gas source is used,
▪ Via the introduction opening (6), the biopharmaceutical contents to be sterilized are introduced directly into the inside receiving space (2),
▪ Then, the introduction opening (6) is closed in an airtight manner,
▪ Then, whereas the sterilization gas conveyance channel (7) is open, the sterilization gas is brought in from the sterilization gas source to the inside receiving space (2) where the biopharmaceutical contents to be sterilized are located, in such a way as to sterilize the biopharmaceutical contents,
▪ Then, the opening/closing means (12) are implemented in such a way as to close the sterilization gas conveyance channel (7); said membrane (9) that is permeable to sterilization gas consists of a permeable zone of the outer wall (3) of the packaging (1) that belongs to a deployable/foldable part of the outer wall (3) of the packaging (1) and that forms the sterilization gas conveyance channel (7), with this deployable/foldable part being capable of being either deployed in such a way that the sterilization gas conveyance channel (7) is open, or folded on itself toward the inside of the packaging (1) in such a way that the sterilization gas conveyance channel (7) is closed,
▪ And thus a sterilized biopharmaceutical packaging (1) that encloses the sterilized biopharmaceutical contents that can undergo one or more integrity tests of the type with differential pressure requiring sealing of the packaging against gas, at any time desired after sterilization, is formed.

12. Process according to Claim 11, wherein:
▪ A biopharmaceutical packaging (1) according to Claim 1 is used, with a membrane (9) that is permeable to sterilization gas constituted by a permeable zone of the outer wall (3) of the packaging (1) belonging to a deployable/foldable part, and opening/closing means (12) that consist of the outer wall (3) whose deployable/foldable part is inserted between two airtight facing side parts are used,
▪ And, after the biopharmaceutical contents have been sterilized, the deployable/foldable part is folded on itself toward the inside of the packaging (1), in such a way that the two facing side parts are located opposite and close together, and they are sealed to one another on their outer periphery in an airtight manner, around the zone that is permeable to the sterilization gas, in such a way as to close the sterilization gas conveyance channel (7).

13. Process according to any one of Claims 11 to 12, wherein once the sterilization is done, the outer wall (3), the sterilization gas conveyance channel (7), and the opening/closing means (12) are kept integral in such a way as to form the sterilized biopharmaceutical packaging (1) that encloses the sterilized biopharmaceutical contents.

14. Process according to Claim 13, wherein once the sterilization is done, the biopharmaceutical packaging (1) is not cut into several segments.

15. Process according to any one of Claims 11 to 14, wherein at any time desired after sterilization, the sterilized biopharmaceutical packaging (1) that encloses the sterilized biopharmaceutical contents is made to undergo one or more integrity tests of the type requiring sealing of the packaging (1) against gas.

16. Sterilized biopharmaceutical packaging (1) that encloses sterilized biopharmaceutical contents, wherein it is obtained by implementing the process according to any one of Claims 11 to 15, with the packaging (1) also comprising an opening for extracting, from the inside to the outside of the packaging (1), the sterilized biopharmaceutical contents, closed in an airtight manner and subsequently capable of being opened in such a way as to extract the biopharmaceutical contents from the inside space (2).
